# EUROPEAN PATENT SPECIFICATION

(11) **EP 1 774 041 B1**
(45) Date of publication and mention of the grant of the patent: **05.10.2011**
(21) Application number: 05804047.8
(22) Date of filing: 03.08.2005
(51) Int. Cl.: C12N 1/06, C12Q 1/68

(54) **Method for obtaining nucleic acids by lysis of microbial samples**
Verfahren zur Gewinnung von Nukleinsäuren durch Lyse mikrobieller Proben
Méthodes permettant d'obtenir des acides nucléiques par lyse d'échantillons microbiens

(30) Priority: 03.08.2004 US 598638 P
(43) Date of publication of application: 18.04.2007
(60) Divisional application: 11163757.5
(73) Proprietor: Novartis Vaccines and Diagnostics, Inc., Emeryville, CA 94608 (US)
(72) Inventor: FONG, Yiu-Lian, Emeryville, CA 94608-2916 (US); TABRIZI, Azita, Emeryville, CA 94608-2916 (US)
(74) Representative: Marshall, Cameron John
(86) International application number: PCT/US2005/027715
(87) International publication number: WO 2006/015374

(56) References cited:
- EP-A- 0 595 167
- WO-A-03/097831
- US-A- 5 776 694
- US-A1- 2004 072 241
- BURIK VAN J-A H ET AL: "COMPARISON OF SIX EXTRACTION TECHNIQUES FOR ISOLATION OF DNA FROM FILAMENTOUS FUNGI" MEDICAL MYCOLOGY, OXFORD, GB, vol. 36, no. 5, 1 October 1998 (1998-10-01), pages 299-303, XP002992578 ISSN: 1369-3786
- VAN BURIK J A ET AL: "Panfungal PCR assay for detection of fungal infection in human blood specimens." JOURNAL OF CLINICAL MICROBIOLOGY MAY 1998, vol. 36, no. 5, May 1998 (1998-05), pages 1169-1175, XP002507508 ISSN: 0095-1137
- GOLBANG N ET AL: "Sensitive and universal method for microbial DNA extraction from blood products." JOURNAL OF CLINICAL PATHOLOGY OCT 1996, vol. 49, no. 10, October 1996 (1996-10), pages 861-863, XP002507617 ISSN: 0021-9746

## Description

### FIELD OF THE INVENTION

The invention relates to simple and rapid methods for isolation of nucleic acids from microbial cells, particularly bacterial and fungal cells, using a universal lysis procedure.

### BACKGROUND OF THE INVENTION

With the advent of molecular biology, an increasing number of diagnostic methods are based on the detection of nucleic acids. Nucleic acid amplification technologies represent useful tools in molecular biology. Since the discovery of the polymerase chain reaction (PCR), various protocols have been described for isolating nucleic acids suitable for detection and identification of microorganisms. However, most of these protocols are time-consuming and often require the use of toxic chemicals. In addition, protocols need to be adapted for each microbe type; a lysis protocol for fungi may not be suitable for gram-negative bacteria, or parasites, or bacterial spores, and so on. Furthermore, these protocols require numerous steps, increasing the risk of sample-to-sample or carry-over contamination. In addition, in many cases the identity of microorganisms present in a sample will be unknown making it impossible to determine which specific protocol would be appropriate or necessitating multiple assays for a single sample.

Many different methods have been described for disrupting microbial cells preliminary to the release of nucleic acid (see, Hugues et al. Methods in Microbiology, 1971, vol. 5B, Academic Press, New York, for an early review of these methods). The method selected will depend on its capability to process samples of a certain size or ability to process multiple samples in a reasonable period of time, while the desired nucleic acids retain their integrity. Classical physical methods of cell breakage include mechanical cell disintegration (crushing and grinding, wet milling, ultrasonics, hydraulic shear, freeze pressure), liquid or hydrodynamic shear (French press, Chaikoff press, homogenizers, wet mills, vibration mills, filters, ultrasonic disintegration) and solid shear (grinding, Hugues press). Chemical and biological methods of cell disintegration are mostly aimed at modifying the cell wall or cytoplasmic membrane, or both, so that the cells either become leaky or burst due to the effects of turgor pressure. Methods include osmosis, drying and extraction, autolysis, inhibition of cell wall synthesis, enzymatic attack on cell walls, bacteriophages and other lytic factors, and ionizing radiation.

Many of the bacterial genomic DNA or plasmid isolation methods use lysozyme or other lytic enzymes (such as mutanolysin or lysostaphin) to achieve enzymatic lysis of bacteria in order to recover the nucleic acid from the cell lysate. Likewise, many of the yeast nucleic acid (genomic DNA or plasmid DNA) isolation methods involve the use of either mechanical shearing force or enzymatic digestion with such as lyticase, zymolase, or chitinase to break down the yeast cell wall to enable the isolation of the nucleic acids. It is known that lysozyme can effectively break down the bacterial cell wall by cleaving the ß-1,4-glycosidic bonds between N-acetylglucosamine and N-acetylmuramic acid in peptidoglycan; the major structure component of the bacterial cell wall. It is also well documented that lyticase can effectively degrade the yeast cell wall mainly via the ß-1,3-glucanse activity. Many of these hydrolytic enzymes are shown to have optimal activity in the presence of divalent metal ions, such as Mg⁺⁺, Ca⁺⁺, etc. It is well documented that lyticase activity requires a reducing agent, such as ß-mercaptoethanol or DTT, to effectively lyse the yeast cell wall. EDTA is a well known metal chelator which is very effective in weakening the outer cell membrane of bacteria by chelating the Mg++ and Ca++, which are essential elements holding the membranes structure by linking LPS (lipopolysaccharide) and proteins together.
WO 03/097831 discloses a method for lysing yeast in which cells are contacted with 0.1MEDTA, 14mM mercaptoethanol and 100U lyticase.

There are a number of kits currently on the market for isolation or detection of nucleic acids from various cellular or viral sources. Typically, a first steps in any method for isolating or detecting nucleic acid from a microbial cell involves the disruption (lysis) of the cell wall and membranes to allow the cellular contents (including the nuclear contents, if present) to be released into the medium. This cell lysis can be accomplished using the lysing enzymes of the types described above. Because of the different requirements of the lysing enzymes used, most of the commercially available kits are specifically designed to isolate nucleic acid from either bacteria or yeast. While there are some kits available that are designed for use with both bacteria and yeast cells, these generally employ mechanical disruption methods (e.g., grinding or vortexing with glass beads) and/or the use of organic solvents for extraction or precipitation, and are therefore not suitable for automation.

There remains a need for a simple, rapid and widely applicable and automatable method for isolating nucleic acid from a variety of microorganisms. Such a method requires a lysis procedure that can be applied to any microorganism present in a sample.

### SUMMARY OF THE INVENTION

The present invention is directed to methods for simple and rapid microbial cell disruption to allow release of microbial nucleic acids in a liquid sample or suspension. The methods of the invention can be applied to many different types of microorganisms, including various bacterial species (both Gram positive and Gram negative), fungal species and viruses, as well as mixtures of these microorganisms. The method is carried out with a minimum of manipulation and, in a preferred embodiment, can be accomplished in a single reaction vessel. The method does not require separate handling for different types of microorganisms. The lysis method of the invention is particularly useful in a method for determining the presence of microbial contamination of a sample, particularly a biological sample (e.g., blood, platelets, concentrated red blood cells, serum, plasma, urine, spinal fluid, tissue fluids, interstitial fluids, saliva, vaginal, dental, uterine or rectal swabs, lavage, sputum, organ or tissue biopsies). Other types of samples are equally suitable for use in the lysis method as long as the sample is known or suspected to contain microorganisms and can be prepared as a liquid sample or suspension (e.g., water samples from a water treatment plant, pharmaceutical solutions or suspensions, swabs from inert surfaces, or processed foods such as milk, fruit juices and other drinks, meats etc.).

The method of the invention as defined in the claims utilizes a mixture of lysing enzymes and a high concentration of a chelating agent to achieve cell disruption of any type of microorganism. No detergent, stabilizing agent, or reducing agent is required to achieve disruption of the microbial cells and allow release of the nucleic acids, although such components may be added. In additional embodiments, the method includes treatment of the lysed microbial cell sample with a protease to reduce the amount of protein in the lysed microbial cell sample and to facilitate the isolation of released nucleic acid. The released nucleic acid may be further isolated by any convenient technique. A particularly preferred technique uses a chaotropic agent, a detergent, and a nucleic acid-binding solid support, and optionally, an alcohol such as ethanol or isopropanol. Various kits are commercially available for this purpose.

Because the method of the invention can be used on a variety of types of microorganisms, can be carried out in a single reaction vessel, and does not require any mechanical or physical shearing methods, it is readily adapted for automated sample handling systems. It is particularly suited for high-throughput automated systems for microbial contamination detection that utilize nucleic acid testing (NAT).

### DETAILED DESCRIPTION

The invention relies in part on the discovery by the present inventors that conventional techniques for microbial cell disruption and concomitant release of nucleic acids and other cellular contents could be simplified into a single, widely applicable procedure suitable for disruption of a wide variety of microorganisms.

The present inventors have discovered conditions that are suitable for the simultaneous enzymatic disruption of both bacterial and yeast cells allowing for the release of nucleic acid from both cell types in a single reaction. The process requires only a high concentration of a chelating agent and a mixture of lysing enzymes suitable for digestion of each cell type (i.e., for bacteria and for yeast). Neither reducing agent (such as β-mercaptoethanol, dithiothreitol or dithioerythritol) nor stabilizing agent (e.g., sorbitol) are required. In some applications, the introduction of a detergent will provide for the solubilization of the cell membranes and facilitate the release of the nucleic acids from the disrupted cells.

The method described herein comprises: providing a liquid sample or suspension for evaluation of possible microbial contamination; producing a sample lysing composition by (1) adding to the sample a chelating agent and (2) adding to the sample a mixture of lysing enzymes; and incubating the sample for sufficient time and at a temperature to produce a lysed microbial sample and thereby allow the release of microbial nucleic acids. In further embodiments, the sample can be treated with a protease to digest any protein present in the lysed microbial sample and the released nucleic acid can additionally be isolated and purified using standard techniques. Further still, purified nucleic acids may be detected and/or analyzed by any conventional detection technique.

By "liquid sample" is meant a sample that is in a liquid state, e.g. a substance in a fluid state with a fixed volume but no fixed shape. Liquid samples can include, but are not limited to, blood, serum, plasma, urine, body fluids, swab samples (obtained by swabbing a surface of interest and placing the swab in common swab media or buffer), buffers or processed fluid food products such as milk, juices or other drinks.

By "suspension" is meant a sample in which particulates are suspended in a liquid and can include, but is not limited to, suspensions of blood cells or other types of cells, or tissue homogenates wherein tissue samples are macerated into aqueous buffers, or suspensions of particulates such as chromatography support material in aqueous buffer solutions.

The liquid sample may be centrifuged for 10 minutes at ≥ 5000 x g and resuspended in a solution containing the chelating agent. In a preferred embodiment, the chelating agent is added to a final concentration between about 0.3 and 0.5 M EDTA. In cases where the sample is already concentrated, such as whole blood, the sample may not need centrifugation and the chelating agent may be added directly into the concentrated sample. The mixture of lysing enzymes is then added and the liquid sample is incubated for a sufficient time and at a sufficient temperature to produce a lysed microbial cell sample wherein the microbial nucleic acids have been released from the microbial cells. In a preferred embodiment, the mixture of lysing enzymes comprises at least one enzyme selected from the group consisting of lysozyme, lysostaphin, and mutanolysin, and at least one enzyme selected from the group consisting of lyticase and zymolyase. In a further preferred embodiment, the sample is incubated for 10 to 60 minutes at a temperature between about 25°C and 37°C. Optionally, a protease (e.g., proteinase K) may be added with or after the addition of the mixture of lysing enzymes. The protease containing lysed microbial sample preferably is incubated between 50°C and 65°C. The method described herein may comprise the further step of isolating released microbial nucleic acid using methods known in the art such as binding of the released microbial nucleic acid to a nucleic-acid binding support (see for example MagAttract DNA or EZ-1 DNA kits by Qiagen, or the Mag DNA Isolation kits by Agowa). Preferably, isolated microbial nucleic acids may then be detected or analyzed using any conventional detection technique known in the art, e.g. amplification techniques such as PCR, TMA, NASBA, RT-PCR, optionally followed by sequence analysis if desired.

The chelating agent is typically provided in a concentrated aqueous solution that is pH-adjusted with small amounts of concentrated acid or base, as appropriate, to achieve a pH in the physiological range. Alternatively, any of several well-known buffers can be used to adjust the pH. The chelating agent will have a pH of about pH 7.0 to about pH 8.0, preferably a pH of about 7.5 +/- 0.1 pH units.

Preferably for the compositions and methods of the present invention, the chelating agent is ethylenediaminetetraacetic acid (EDTA) or ethylene glycol-bis(2-aminoethylether) tetraacetic acid (EGTA), or their salts; more preferably, the chelating agent is EDTA. The terms "EDTA" and EGTA" will be used to refer both to the acid and the salt form, and either form may be used in the present invention, although the salt forms are preferred.

By "high concentration of a chelating agent" is meant a final concentration of at least 0.3M to at least 0.5M. Preferably the chelating agent is present in methods of the inventions at a concentration of between 0.4M and 0.5M.

By "lysing enzyme" is meant any of a number of well-known enzymes that act to digest components of microbial cell walls, thus causing the cell to be disrupted or lyse. Examples of lysing enzymes include, but are not limited to, lyticases, chitinases, zymolases, gluculases, lysozymes, lysostaphins, and mutanolysins. In one embodiment, the mixture of lysing enzymes in the present composition and methods will comprise at least one lysing enzyme having glucanase activity against fungal (e.g., yeast) cell walls and at least one enzyme having glycolytic activity against bacterial cell wall peptidoglycans. In another embodiment, the mixture of lysing enzymes will comprise at least one enzyme selected from the group consisting of a lyticase, a zymolase, and hydrolytic enzymes having β-1,3-glucanase activity, β-1,4-glucanase activity or β-1,6-glucanase activity, and at least one enzyme selected from the group consisting of a lysozyme, a mutanolysin and a lysostaphin. In a preferred embodiment, the lysing composition of the invention consists essentially of a high concentration of a chelating agent and a mixture of a lyticase and/or a zymolase combined with a lysozyme and/or a mutanolysin and/or a lysostaphin. All of these enzymes are well known and readily available from a variety of commercial sources.

The addition of detergent is optional and may be accomplished before, after or with the addition of chelating agent and/or the mixture of lysing enzymes. Any of several well-known detergents for solubilizing the cell membrane are suitable, including but not limited to, Triton X-100, Tween 20, NP-40, and SDS.

In another aspect, the present invention provides a method for microbial cell disruption to allow release of nucleic acid from microbial cells present in a sample as defined in the claims comprising: providing a sample containing or suspected of containing microbial cells, wherein the sample is a liquid sample or suspension, and producing a sample lysing composition by (1) adding a chelating agent to the sample to a final concentration of between 0.3M and 0.5M, (2) adding to the sample a mixture of lysing enzymes, and incubating the sample for sufficient time and at a temperature to produce a lysed microbial sample and thereby to allow the release of the microbial nucleic acids. The chelating agent and the mixture of lysing enzymes are as described above for the lysing composition. Typically, the sample lysing composition will contain only the sample, the chelating agent and the mixture of lysing enzymes and typically will not contain stabilizing agent or reducing agent. The sample lysing composition typically will not contain any added divalent metal cations (although some small amount may be present in the samples initially). The chelating agent and the lysing enzyme mixture may be added to the sample sequentially in any order or may be added simultaneously. The chelating agent and the lysing enzyme mixture may be combined and added to the sample in a single step. The final concentration of chelating agent in the sample lysing composition will be between 0.3 M and 0.5M; most preferably, the final concentration of chelating agent in the sample lysing composition will be between about 0.4M and about 0.5M. The preferred chelating agents are EDTA and EGTA. In some embodiments, the sample lysing composition may contain a detergent (e.g., Triton X-100, Tween 20, NP-40, or SDS) to facilitate solubilization of the cell membranes.

In a preferred embodiment, the mixture of lysing enzymes comprises, preferably consists essentially of, a lyticase and/or a zymolase and a lysozyme and/or a mutanolysin and/or a lysostaphin. Thus, preferred lysing enzyme mixtures contain lyticase and lysozyme; or lyticase and mutanolysin; or lyticase and lysostaphin; or zymolase and lysozyme; or zymolase and mutanolysin; or zymolase and lysostaphin; or lyticase, zymolase and lysozyme; or lyticase, zymolase and mutanolysin; or lyticase, zymolase and lysostaphin; or lyticase, lysozyme and mutanolysin; or zymolase, lysozyme and mutanolysin; or lyticase, lysozyme, lysostaphin and mutanolysin; or zymolase, lysozyme, lysostaphin and mutanolysin; or lyticase, zymolase, lysozyme and mutanolysin; or lyticase, zymolase, lysozyme, lysostaphin and mutanolysin, etc.

As defined in the claims, the method comprises: providing a liquid sample or suspension containing or suspected of containing microbial cells; producing a sample lysing composition by (1) adding a chelating agent to the sample to a final concentration of between about 0.3M and 0.5M, and (2) adding to the sample a mixture of lysing enzymes; and incubating the sample for sufficient time and at a temperature to produce a lysed microbial sample and thereby to allow the release of microbial nucleic acids.

The method may comprise: providing a liquid sample or suspension containing or suspected of containing microbial cells; producing a sample lysing composition by (1) adding a chelating agent to the sample to a final concentration of between about 0.3M and 0.5M, and (2) adding to the sample a mixture of lysing enzymes, wherein the mixture of lysing enzymes consists essentially of at least one enzyme selected from the group consisting of lyticase and zymolase, and at least one enzyme selected from the group consisting of lysozyme, lysostaphin and mutanolysin; and incubating the sample for sufficient time and at a temperature to produce a lysed microbial sample and thereby to allow the release of the microbial nucleic acids.

The incubation time and temperature conditions sufficient to produce a lysed microbial sample will be readily determined be one of ordinary skill in the art and will depend in part on the requirements of the particular lysing enzymes chosen. In general, the time of the incubation step is about 10 to about 60 minutes, preferably between 30 and 60 minutes, at temperatures between about 25° C and about 37° C, preferably between 30-37° C, will be suitable.

In a further embodiment, the method comprises: providing a liquid sample or suspension containing or suspected of containing microbial cells; producing a sample lysing composition by (1) adding a chelating agent to the sample to a final concentration of between 0.3M and 0.5M, and (2) adding to the sample a mixture of lysing enzymes, wherein the mixture of lysing enzymes consists essentially of at least one enzyme selected from the group consisting of lyticase and zymolase, and at least one enzyme selected from the group consisting of lysozyme and mutanolysin; and incubating the sample for between about 10 minutes and about 60 minutes and at a temperature of between about 25° C and about 37°C to produce a lysed microbial sample and thereby to allow the release of microbial nucleic acids.

The lysing enzymes will be present in the lysing composition and the sample lysing composition at concentrations sufficient to achieve lysis of microbial cells present in the sample. The appropriate concentrations are readily determined by one of ordinary skill in the art and typically will range from 0.1 unit/mL to 10⁶ units/mL. However, it will be readily apparent to one of ordinary skill in the art that parameters of enzyme concentration, incubation time and incubation temperature, are interdependent and can be adjusted in various ways to achieve the same or very similar result. For example, a lower enzyme concentration can be compensated for by a longer incubation time, a lower incubation temperature can be compensated for by a longer incubation time and/or a higher enzyme concentration.

In a further aspect, the method of the invention additionally comprises the step of adding a protease; preferably, a non-specific protease; more preferably, a proteinase K, to the lysed microbial sample and incubating to digest any protein present in the lysed microbial cell sample. The protease can be added to the sample after the chelating agent and mixture of lysing enzymes or simultaneously with the chelating agent or the mixture of lysing enzymes. In order to accomplish digestion of the protein present, the lysed microbial sample with the added protease will be incubated at a temperature and for a time sufficient to allow the protease to work. These conditions are well known and readily determined by one of ordinary skill in the art. Typically, the protease-containing lysed microbial sample will be incubated for about 10 minutes to about 60 minutes at a temperature appropriate for the protease used. In particular, when proteinase K is used, the lysed microbial sample will be incubated at between about 50° C and about 65° C.

Following protease digestion, the released nucleic acids can optionally be isolated using any convenient technique (see, e.g., US Patents 5,234,809; 6,465,639; 6,673,631; 6,027,945; 6,383,393; 5,945,525; 6,582,922, inter alia). A number of kits/reagents are available commercially for carrying out nucleic acid isolation, for example, the MagAttract DNA kits or EZ-1 DNA kits from Qiagen (Valencia CA, catalogue number 953336) and the Mag DNA Isolation kits from Agowa (Berlin, Germany, catalogue number 953034). These kits utilize silica-based magnetic beads and chaotropic agents to non-specifically bind nucleic acid to the beads. Any silica membrane based methods can also be used, such as QIAamp DNA kits (Qiagen, for example catalogue numbers 51304, 51161, 51192, 51104, 52904) and Nucleospin kits (Machery-Nagel, for example catalogue numbers 740951, 740691, 740740, 740623,). Other suitable kits include the Magnesil or the 96 Wizard kits (Promega, catalogue number A2250), the Nucleomag kit (Machery-Nagel, catalogue number 744500), DNA Direct kit (Dynal, catalogue number 630.06) and Magnazorb (Cortex Biochem., catalogue numbers MB1001, MB2001) The supplier's protocols are followed when using these kits except that the steps and reagents for cell-wall lysis, if any are included, are replaced by the lysis methods of the present invention.

The isolated nucleic acids can be detected and/or analyzed by any conventional detection technique, including e.g., amplification techniques such as PCR, TMA, NASBA, RT-PCR, optionally followed by sequencing analysis, if it is desirable for determination of the types, species and strains of microorganism detected. The target for amplification and detection can be one that is similar among a wide variety of microbial species (e.g., 16S RNA gene, 23S RNA gene, tuf (elongation factor Tu) gene, or any conserved housekeeping gene for bacteria or yeast) or can be one that is specific for a particular organism.

The lysis method of the invention is carried out on a sample that contains or is suspected to contain microbial cells or microorganisms. The terms "microbial cells" and "microorganisms" are used interchangeably and refer to any single- celled organism, whether prokaryotic or eukaryotic. Typically, the samples used in the lysis method will contain the microorganisms in very low concentrations, e.g., as a contaminant. The sample may contain other types of cells (e.g., single cells or multicellular particles from a multicellular organism (e.g., a human blood sample).

By "lysis" of a microbial cell is intended the disruption, rupture, poration, permeabilization, digestion or break down of the microbial cell wall such that the nucleic acid components of the cell can be released into the external medium. In some applications, the release of the nucleic acids into the external medium may be facilitated by the addition of detergent that acts to solubilize the cell membranes. According to the invention, the microbial cell wall need not be completely disrupted, ruptured, permeabilized or digested in order to effect the release of the nucleic acids.

By "release" of the microbial nucleic acids is intended that the microbial nucleic acids, particularly the genomic nucleic acids, are no longer retained within the cell but are free and accessible to various nucleic acid isolation procedures.

By "inert surface" is meant any number of solid surfaces wherein suspected microbial contamination may have occurred. Such surfaces can include, but are not limited to, a laboratory bench, surfaces found in a hospital setting such as walls or tables, or surfaces or machines common to those in the food preparation or manufacturing industry.

The following examples are illustrative of this invention. They are not intended to be limiting upon the scope thereof.

### EXAMPLES

EXAMPLE 1. Titration of chelating agent for microbial lysis

For these examples, the following microorganisms were used: *C. albicans*: ATCC 14053-U; *B. cereus*: ATCC 14579; *K. oxytoca*: ATCC 33496; *S*. *aureus*: ATCC 6538; and *S*. *agalactiae*: ATCC 12386. PCR-based detection of these organisms was performed using the target genes listed as shown: *C. albicans*: tuf (elongation factor Tu), *B. cereus*: 16S rRNA, *K. oxytoca*: 23S rRNA, *S*. *aureus*: 23S rRNA, and *S*. *agalactiae*: cfb (CAMP factor).

To evaluate and optimize the effect of the chelating agent EDTA, used in combination with lyticase and lysozyme in the sample lysing composition, the following protocol was employed. *C. albicans* and *B. cereus* from logarithmically growing cultures were spiked into 2 mL platelet samples (platelets used were purchased from blood banks as either platelets prepared via aphoresis or as random donor platelet samples) at 243 and 24 CFU/mL for *B. cereus,* and 155 and 16 CFU/mL for *C*. *albicans.* CFU/mL calculations were made according to previously established CFU/mL to OD600 nm correlations. Samples were centrifuged at 5000 x g for 10 minutes and the resulting pellets were resuspended in 150 µL of EDTA-containing solution. For this study, EDTA-containing solutions were made up ranging from 0.2 to 0.5 M EDTA, pH 7.5, where the EDTA solutions varied in 0.05 M concentration increments. Freshly prepared lysozyme (10 µL at 400 mg/mL,) (Sigma catalogue number L-7651) and lyticase (10 µL at 1000 units/mL) (Sigma catalogue number L-2425) were added and the mixture was incubated for 60 minutes at 37°C, followed by proteinase K addition (10 µL at 600 mAu/mL) (Qiagen, catalogue number 19131) and a second incubation at 55°C for 60 minutes. Following enzymatic digestion, samples were processed using the reagents in the Qiagen MagAttract DNA Mini M48 Kit (Qiagen, catalog 953336) in combination with an Agowa magnetic separator (MaxiSep 7200, catalog number 4040). Briefly, following enzymatic digestion, samples were mixed with 720 µL of Qiagen MTL lysis buffer from the above-described kit, and incubated for 5 minutes at room temperature. 30 µL of homogeneously suspended MagAttract Suspension Beads from the kit were then added to each sample and mixed for 5 minutes. Supernatant was removed by using the Agowa separator and discarded, while the beads were washed in the separator for 2 minutes using 325 µL of buffer MW1 from the Qiagen kit. A second aliquot of 325 µL of MW1 buffer was added to the beads, and the beads were washed for additional 2 min. Next, the beads were similarly washed twice with buffer MW2 from the Qiagen kit, and lastly rinsed with 650 µL of Qiagen Rinse Buffer or dH₂O. Bound genomic DNA was eluted from the beads by cycling the beads in the Agowa separator in 100 µL of dH₂O at 65°C for 2 minutes. Eluates (10 µL) were tested by PCR using an instrument designed to assay PCR product production in real time (for example My iQ (BIORAD)) using SYBR Green incorporation (Molecular Probes, Oregon). All PCR assays were performed in duplicate and PCR positive results were verified for correct product formation by melting curve analysis. In this analysis, all PCR reactions positive for product formation by an increase in SYBR Green fluorescence were subject to melting temperature analysis wherein the melting temperatures (measured as a loss of SYBR Green fluorescence) in each reaction were observed and compared to the known characteristic melting temperature for each specific target. The cycle threshold (Ct) values (defined as the number of PCR cycles required to measure a specified increase in the SYBR Green fluorescence relative to baseline, indicating specific product formation) from each of the run replicates were averaged, and are depicted in Table 1 below. These experiments indicate that a preferred EDTA concentration is from about 0.3 and 0.5 M. Control experiments, using only the above described DNA isolation kits, and wherein microbial samples were not treated with chelating agent nor with lytic enzymes, were far less sensitive and were only able to detect microbes at much higher spike concentrations.

| *C. albicans* | 155 CFU/mL | | | 16 CFU/mL | | |
|---|---|---|---|---|---|---|
| EDTA Concentration | Average CT | %CV | SD | Average CT | %CV | SD |
| 0.50 M | 32.5 | 1.9 | 0.62 | 35.0 | 1.9 | 0.7 |
| 0.45 M | 33.2 | 2.2 | 0.72 | 35.6 | 3.3 | 1.2 |
| 0.40 M | 34.5 | 1.9 | 0.64 | 35.9 | 2.2 | 0.8 |
| 0.35 M | 34.6 | 3.7 | 1.31 | 36.4^{b} | 2.9 | 1.1 |
| 0.30 M | 36.6 | 2.6 | 0.95 | 38.0^{a} | 4.0 | 1.5 |
| 0.25 M | 37.0^{a} | N/A | N/A | N/A^{c} | N/A | N/A |
| 0.2 M | 38.9^{b} | N/A | N/A | N/A^{c} | N/A | N/A |

**Table 1 EDTA concentrations and C. albicans and B. cereus**

| *B. cereus* | 243 CFU/mL | | | 24 CFU/mL | | |
|---|---|---|---|---|---|---|
| EDTA Concentration | Average CT | %CV | SD | Average CT | %CV | SD |
| 0.50 M | 25.3 | 2.8 | 0.71 | 28.1 | 1.38 | 0.39 |
| 0.45 M | 25.3 | 1.3 | 0.32 | 29.0 | 0.55 | 0.16 |
| 0.40 M | 26.0 | 0.5 | 0.13 | 28.4 | 1.99 | 0.56 |
| 0.35 M | 25.2 | 1.9 | 0.47 | 28.4 | 1.31 | 0.37 |
| 0.30 M | 25.4 | 1.3 | 0.33 | 28.1 | 1.18 | 0.33 |
| 0.25 M | 25.3 | 0.6 | 0.14 | 28.3 | 1.43 | 0.40 |
| 0.2 M | 25.5 | 0.05 | 0.01 | 29.0 | 0.62 | 0.18 |

| | | | | | | |
|---|---|---|---|---|---|---|
| a- 1 in 4 PCR reactions produced positive results b- 2 in 4 PCR reactions produced positive results c- No positive results obtained from 4 PCR reactions | | | | | | |

EXAMPLE 2- Determination of enzymatic digestion temperature

To evaluate preferred incubation temperatures for the lytic enzymes, the following protocol was followed wherein the temperature used during the incubation with lyticase and lysozyme was carried out for 60 minutes at either 30°C or 37°C. This incubation was followed by incubation at 55°C, designed for optimal proteinase K digestion activity. Briefly, log phase *C*. *albicans* or *B. cereus* (100 CFU/mL) were spiked into 3 mL platelet samples. Samples were centrifuged at 5000 x g for 10 minutes and pellets were resuspended in 200 µL of 0.5 M EDTA, pH 7.5. Lyticase (10 µL, 1000 units/mL), lysozyme (10 µL, 400 mg/mL) and proteinase K (10 µL, 600 mAu/mL) were added to the resuspended samples. The samples were then incubated for 60 minutes at 30°C or 37°C, followed by a proteinase K incubation at 55°C for 60 minutes. At the conclusion of the enzymatic digestions, the samples were processed for PCR as described in Example 1. Table 2 depicts the results from this evaluation and indicates that 37°C can be a preferred incubation temperature.

**Table 2- Incubation temperatures and C. albicans or B. cereus**

| | ***C. albicans*** | | | ***B. cereus*** | | |
|---|---|---|---|---|---|---|
| Incubation temperature | Average CT | %CV | SD | Average CT | %CV | SD |
| 30°C | 30.2 | 3.8 | 1.1 | 27.3 | 1.9 | 0.5 |
| 37°C | 29.1 | 5.2 | 1.5 | 26.5 | 1.5 | 0.4 |

EXAMPLE 3- Determination of incubation time for enzymatic digestion

To evaluate a minimum incubation time for each of the two enzymatic digestions: (1) 37°C for lyticase, lysozyme and (2) 55°C for proteinase K that will allow for efficient processing of the samples, incubation times at each temperature were varied between 15 and 45 minutes. Log phase microorganisms were spiked into 3 mL platelet samples that were then centrifuged at 5000 x g for 10 minutes. The resultant pellets were resuspended in 150 µL of 0.5 M EDTA, pH 7.5. Lyticase (10 µL, 1000 units/mL), lysozyme (10 µL, 400 mg/mL) and proteinase K (20 µL, 600 mAu/mL) were added to the resuspended samples, and the samples were incubated at 37°C for the specified period. Following the 37°C incubation, samples were incubated at 55°C for the specified time. Samples were then processed as described in Example 1, and 5 µL eluate samples were evaluated by PCR. The data is presented below in Table 3, and indicated that a preferred incubation scheme was 37°C for 30 minutes, followed by 55°C for 30 minutes. For *C*. *albicans* at 10 CFU/mL, only the 15 min at 37°C, 15 at 55°C incubation test gave positive PCR results.

| Incubation temp, time | | ***B. cereus*** | | | | ***K. oxytoca*** | | | |
|---|---|---|---|---|---|---|---|---|---|
| | | 118 CFU/mL | | 11.8 CFU/mL | | 132 CFU/mL | | 13.2 CFU/mL | |
| 37°C | 55°C | CT | SD | CT | SD | CT | SD | CT | SD |
| 15 min | 15 min | 30.6 | 0.8 | 33.8 | 1.4 | 26.4 | 1.0 | 29.5 | 0.1 |
| 15 | 30 | 28.9 | 0.4 | 32.8 | 1.6 | 26.0 | 0.3 | 29.2 | 0.5 |
| 30 | 15 | 28.3 | 0.5 | 31.7 | 1.5 | 25.9 | 0.5 | 28.1 | 0.7 |
| 30 | 30 | 26.0 | 0.6 | 31.1 | 0.9 | 26.0 | 0.5 | 28.9 | 0.4 |
| 30 | 45 | 25.7 | 0.5 | 31.7 | 1.1 | 26.0 | 0.4 | 28.8 | 0.2 |
| 20 | 20 | 28.2 | 0.2 | 31.6 | 1.0 | 26.0 | 0.4 | 28.5 | 0.1 |
| 30 | 60 | 28.1 | 1.1 | 31.6 | 0.4 | 26.0 | 0.5 | 28.8 | 0.1 |

| Incubation temp, time | | ***S. aureus*** | | | | ***S. agalactiae*** | | | |
|---|---|---|---|---|---|---|---|---|---|
| | | 56 CFU/mL | | 5.6 CFU/mL | | 136.5 CFU/mL | | 13.7CFU/mL | |
| 37°C | 55°C | CT | SD | CT | SD | CT | SD | CT | SD |
| 15 min | 15 min | 31.9 | 0.4 | 36.0 | 2.1 | 28.8 | 0.3 | 32.8 | 0.8 |
| 15 | 30 | 30.7 | 0.6 | 32.6 | 0.7 | 27.9 | 0.4 | 30.9 | 0.6 |
| 30 | 15 | 30.6 | 0.4 | 36.1 | 1.5 | 27.7 | 0.8 | 29.9 | 1.2 |
| 30 | 30 | 29.0 | 1.0 | 32.3 | 3.8 | 27.3 | 0.2 | 32.0 | 0.6 |
| 30 | 45 | 29.9 | 0.3 | 36.3 | 1.6 | 27.3 | 0.3 | 30.8 | 0.6 |
| 20 | 20 | 30.2 | 0.5 | 32.5 | 2.3 | 27.3 | 0.2 | 30.8 | 0.6 |
| 30 | 60 | 32.6 | 1.1 | 32.6 | 1.3 | 28.0 | 0.3 | 30.1 | 0.1 |

**Table 3- Incubation times and B. cereus, K. oxytoca, S. aureus, S. agalactiae, or C. albicans**

| Incubation temp, time | | ***C. albicans*** | | | |
|---|---|---|---|---|---|
| | | 100 CFU/mL | | 10 CFU/mL | |
| 37°C | 55°C | CT | SD | CT | SD |
| 15 min | 15 min | 37.4 | 2.4 | 37.2 | 0.6 |
| 15 | 30 | 33.5 | 1.4 | ND | ND |
| 30 | 15 | 36.1 | 1.4 | ND | ND |
| 30 | 30 | 33.1 | 1.5 | ND | ND |
| 30 | 45 | 32.1 | 1.1 | ND | ND |
| 20 | 20 | 34.3 | 0.5 | ND | ND |
| 30 | 60 | 33.5 | 0.6 | ND | ND |

EXAMPLE 4- Evaluation of methodology in a variety of samples

A variety of sample matrices were evaluated in the method of the invention. In each case, microorganisms were spiked into the various sample types, and then the protocol followed to determine if the spiked microorganisms could be detected. In the first data set, whole blood, plasma and urine samples were evaluated, and in the second set, swab samples from skin, mouth and lab bench were spiked with the test microorganisms and processed.

For the evaluation of the protocol in whole blood, plasma and urine, the following protocol was followed: 3 mL plasma and urine sample volumes were spiked with test microorganisms, while with the whole blood samples, 0.1 mL volumes were spiked. The samples were centrifuged and resuspended as described in Example 1 with the exception that the whole blood sample was not centrifuged. The samples were processed as described, using 30 minutes at 37°C and 30 minutes at 55°C for the enzyme incubation times. 5 µL eluate samples were subject to PCR as described in Example 1, and the data is presented below in Table 4.

| ***B. cereus*** | | | | | |
|---|---|---|---|---|---|
| Sample | CFU/mL | CT | SD | %CV | PCR + |
| 0.1 mL blood | 40 | 35.22 | ND | ND | 2/6 |
| | 80 | 34.54 | ND | ND | 5/6 |
| | 240 | 32.92 | 1.68 | 5.12 | 6/6 |
| | 15 | 31.52 | 0.97 | 3.06 | 6/6 |
| 3 mL plasma | 8 | 32.37 | ND | ND | 5/6 |
| | 24 | 31.49 | 0.99 | 3.14 | 6/6 |
| | 80 | 29.58 | 0.94 | 3.18 | 6/6 |
| | 240 | 26.12 | 0.13 | 0.52 | 6/6 |
| 3 mL urine | 8 | 34.63 | ND | ND | 2/6 |
| | 24 | 33.73 | ND | ND | 3/6 |
| | 80 | 33.07 | 1.17 | 3.54 | 6/6 |
| | 240 | 30.89 | 1.49 | 4.82 | 6/6 |

| ***K. oxytoca*** | | | | | |
|---|---|---|---|---|---|
| Sample | CFU/mL | CT | SD | %CV | PCR + |
| 0.1 mL blood | 76 | 33.78 | 2.06 | 6.10 | 6/6 |
| | 153 | 31.35 | 1.21 | 3.86 | 6/6 |
| | 458 | 30.14 | 0.74 | 2.45 | 6/6 |
| | 1525 | 28.49 | 0.64 | 2.25 | 6/6 |
| 3 mL plasma | 15 | 29.65 | ND | ND | 5/6 |
| | 46 | 28.07 | 0.43 | 1.54 | 6/6 |
| | 153 | 26.31 | 0.58 | 2.21 | 6/6 |
| | 458 | 24.18 | 0.33 | 1.36 | 6/6 |
| 3 mL urine | 15 | 30.02 | 0.56 | 1.88 | 6/6 |
| | 46 | 28.55 | ND | ND | 5/6 |
| | 153 | 26.90 | 0.30 | 1.10 | 6/6 |
| | 458 | 25.83 | ND | ND | 5/6 |

| ***S. aureus*** | | | | | |
|---|---|---|---|---|---|
| Sample | CFU/mL | CT | SD | %CV | PCR + |
| 0.1 mL blood | 11 | 34.42 | ND | ND | 5/6 |
| | 22 | 33.50 | ND | ND | 5/6 |
| | 68 | 33.26 | ND | ND | 5/6 |
| | 225 | 32.93 | 1.89 | 5.75 | 6/6 |
| 3 mL plasma | 2 | 34.86 | ND | ND | 3/6 |
| | 7 | 34.12 | 1.93 | 5.66 | 5/6 |
| | 23 | 32.73 | 0.97 | 2.96 | 6/6 |
| | 68 | 30.29 | 0.76 | 2.51 | 6/6 |
| 3 mL urine | 2 | 35.79 | ND | ND | 5/6 |
| | 7 | 35.86 | ND | ND | 3/6 |
| | 23 | 33.78 | 1.42 | 4.20 | 5/6 |
| | 68 | 32.85 | 1.01 | 3.09 | 6/6 |

| ***S. agalactiae*** | | | | | |
|---|---|---|---|---|---|
| Sample | CFU/mL | CT | SD | %CV | PCR + |
| 0.1 mL blood | 39 | 36.98 | ND | ND | 4/6 |
| | 78 | 32.88 | 1.28 | 3.88 | 6/6 |
| | 234 | 31.52 | 1.16 | 3.69 | 6/6 |
| | 780 | 30.37 | 1.01 | 3.33 | 6/6 |
| 3 mL plasma | 7.8 | 31.59 | 0.85 | 2.68 | 6/6 |
| | 23.4 | 30.90 | 0.24 | 0.77 | 6/6 |
| | 78 | 28.50 | 0.72 | 2.53 | 6/6 |
| | 234 | 26.36 | 0.82 | 3.12 | 6/6 |
| 3 mL urine | 7.8 | 32.35 | 0.95 | 2.94 | 6/6 |
| | 23.4 | 31.71 | ND | ND | 5/6 |
| | 78 | 29.38 | 1.04 | 3.55 | 6/6 |
| | 234 | 28.05 | 0.75 | 2.68 | 6/6 |

**Table 4- Detection of microorganisms in whole blood, plasma or urine**

| ***C. albicans*** | | | | | |
|---|---|---|---|---|---|
| Sample | CFU/mL | CT | SD | %CV | PCR + |
| 0.1 mL blood | 30.5 | ND | ND | ND | 0/6 |
| | 61 | 29.81 | ND | ND | 4/6 |
| | 183 | 26.18 | 4.82 | 18.42 | 6/6 |
| | 610 | 32.50 | ND | ND | 4/6 |
| 3 mL plasma | 6.1 | 28.43 | ND | ND | 4/6 |
| | 18.3 | 30.89 | 3.02 | 9.78 | 6/6 |
| | 61 | 28.70 | 4.00 | 13.94 | 6/6 |
| 3 mL urine | 6.1 | 25.73 | 5.72 | 22.24 | 6/6 |
| | 18.3 | 23.21 | ND | ND | 2/6 |
| | 61 | 27.35 | ND | ND | 4/6 |

To evaluate the protocol for the detection of microorganisms in swab samples, test microorganisms were spiked into swab samples collected from skin, mouth or a lab bench. For these experiments, two types of common clinical swab media were used: Amies medium (3.0 g NaCl, 0.2 g KCl, 0.1 g CaCl, 0.1 g MgCl, 0.2 g monopotassium phosphate, 1.15 g disodium phosphate, 1.0 g sodium thioglycollate per liter) (LQ Amies Swabs from Health Link, catalogue number 4140 BX.) or Stuart medium (10.0 g sodium glycerophosphate, 0.1 g. CaCl, 1.0 mL mercaptoacetic acid per liter) (CultureSwab™ Liquid Stuart from BD Diagnostics, catalogue number 220109). 0.1 mL of swab medium was placed in swab tubes, and the swab samples were taken by swabbing either skin, mouth or lab bench surfaces with the swabs. Samples were incubated in the original swab tubes for at least 5 minutes. Test samples were then prepared by transferring the swabs to tubes containing 0.2 mL PBS spiked with the test microorganisms. All swabs were taken in duplicate and processed according to the protocol described in Example 1, with the incubation conditions being those described in Example 3. 5 µL eluate samples were used for PCR analysis, and the data is presented below in Table 5. The results indicate that the method can be capable of detecting microorganisms in swab samples.

| ***B. cereus*** | | | | | | |
|---|---|---|---|---|---|---|
| Sample | Swab source | CFU/mL | CT | SD | %CV | PCR + |
| Stuart medium | Mouth | 1070 | 27.43 | 0.45 | 1.64 | 4/4 |
| | Skin | 1070 | 27.55 | 0.49 | 1.77 | 4/4 |
| | Bench | 1070 | 27.19 | 0.26 | 0.94 | 4/4 |
| Amies medium | Mouth | 1070 | 27.77 | 0.32 | 1.16 | 4/4 |
| | Skin | 1070 | 27.04 | 0.57 | 2.13 | 4/4 |

| ***C. albicans*** | | | | | | |
|---|---|---|---|---|---|---|
| Sample | Swab source | CFU/mL | CT | SD | %CV | PCR + |
| Stuart medium | Mouth | 530 | 31.39 | 0.34 | 1.08 | 4/4 |
| | Skin | 530 | 31.16 | 0.32 | 1.04 | 4/4 |
| | Bench | 530 | 31.52 | 0.56 | 1.78 | 4/4 |
| Amies medium | Mouth | 530 | 31.07 | 0.55 | 1.78 | 4/4 |
| | Skin | 530 | 31.39 | 0.46 | 1.45 | 4/4 |

**Table 5- Detection of spiked microorganisms in swab samples**

| ***S. agalactiae*** | | | | | | |
|---|---|---|---|---|---|---|
| Sample | Swab source | CFU/mL | CT | SD | %CV | PCR + |
| Stuart medium | Mouth | 1365 | 28.87 | 0.74 | 2.55 | 4/4 |
| | Skin | 1365 | 28.88 | 1.01 | 3.50 | 4/4 |
| | Bench | 1365 | 29.36 | 0.71 | 2.42 | 4/4 |
| Amies medium | Mouth | 1365 | 29.59 | 0.46 | 1.56 | 4/4 |
| | Skin | 1365 | 29.74 | 0.40 | 1.35 | 4/4 |

## Claims

1. A method of microbial cell disruption to allow release of nucleic acid from microbial cells present in a sample comprising:
providing a sample containing or suspected of containing microbial cells, wherein the sample is a liquid sample or suspension,
producing a sample lysins composition by (1) adding a chelating agent to the sample to a final concentration of between 0.3 M and 0.5 M (2) adding to the sample a mixture of lysing enzymes,
and incubating the sample for sufficient time and at a temperature to produce a lysed microbial cell sample and to thereby allow the release of the microbial nucleic acid.

2. The method of microbial cell disruption according to claim 1, wherein said chelating agent is selected from the group consisting of EDTA and EGTA and their salts.

3. The method of microbial cell disruption according to any one of the proceeding claims, wherein said mixture of lysing enzymes comprises at least one enzyme selected from the group consisting of lysozyme, lysostaphin, and mutanolysin, and at least one enzyme selected from the group consisting of lyticase and zymolase.

4. The method of microbial cell disruption according to any one of the proceeding claims, wherein said mixture of lysing enzymes consists essentially of at least one enzyme selected from the group consisting of lysozyme, lyostaphin and mutanolysin, and at least one enzyme selected from the group consisting of lyticase and zymolase.

5. The method of microbial cell disruption according to any one of the proceeding claims, wherein said incubation is at between about 25° C and about 37° C for between about 10 minutes and about 60 minutes.

6. The method of microbial cell disruption according to any one of the proceeding claims, wherein said chelating agent and said mixture of lysing enzymes are added to the sample at the same time.

7. The method of microbial cell disruption according to any one of the proceeding claims, wherein said chelating agent is EDTA.

8. The method of microbial cell disruption according to any one of the proceeding claims, further comprising making a protease-containing lysed microbial sample by adding a protease to said liquid sample or suspension.

9. The method of microbial cell disruption according to claim 8, further comprising:
incubating said protease-containing lysed microbial sample at a temperature of between about 50° C and about 65° C.

10. The method of microbial cell disruption of claim 8 or claim 9, wherein said protease is added to said lysed microbial cell sample.

11. The method of microbial cell disruption of claim 8, 9 or 10, wherein the protease is added to said liquid sample or suspension at the same time as said mixture of lysing enzymes.

12. The method of microbial cell disruption of claim 8, 9, 10 or 12, wherein said protease is proteinase K.

13. The method of microbial cell disruption of any one of claims 1-12, comprising the further step of isolating said released microbial nucleic acids.

14. The method of claim 13, wherein said isolating step comprises adding a chaotropic agent, a detergent and a nucleic acid-binding support to said lysed microbial cell sample under conditions that allow binding of said released nucleic acids to said nucleic acid-binding support.

15. The method of any one of claims 1-14, wherein said sample is selected from the group consisting of a blood sample, a concentrated red blood cell sample, a platelet sample, a plasma sample, a serum sample, a urine sample, a saliva sample, a spinal fluid sample, an interstitial fluid sample, a tissue biopsy sample, a lavage sample, a sputum sample, and a vaginal, dental, rectal, uterine or inert surface swab sample.

## Patentansprüche

1. Verfahren zum mikrobiellen Zellaufschluss, das die Freisetzung von Nukleinsäure aus in einer Probe vorhandenen mikrobiellen Zellen gestattet und Folgendes umfasst:
Bereitstellen einer Probe, die mikrobielle Zellen enthält oder vermutlich enthält, wobei es sich bei der Probe um eine Flüssigkeitsprobe oder Suspension handelt;
Herstellen einer Probenlysezusammensetzung durch (1) Zugabe eines Chelatbildners zu der Probe auf eine Endkonzentration zwischen 0,3 M und 0,5 M, (2) Zugabe eines Gemischs von Lyseenzymen zu der Probe;
und Inkubieren der Probe über einen hinreichend langen Zeitraum und bei einer Temperatur, so dass eine lysierte mikrobielle Zellprobe produziert und damit die Freisetzung der mikrobiellen Nukleinsäure gestattet wird.

2. Verfahren zum mikrobiellen Zellaufschluss nach Anspruch 1, wobei der Chelatbildner ausgewählt ist aus der Gruppe bestehend aus EDTA und EGTA und ihren Salzen.

3. Verfahren zum mikrobiellen Zellaufschluss nach einem der vorhergehenden Ansprüche, wobei das Gemisch von Lyseenzymen wenigstens ein Enzym, ausgewählt aus der Gruppe bestehend aus Lysozym, Lysostaphin und Mutanolysin, und wenigstens ein Enzym, ausgewählt aus der Gruppe bestehend aus Lyticase und Zymolase, umfasst.

4. Verfahren zum mikrobiellen Zellaufschluss nach einem der vorhergehenden Ansprüche, wobei das Gemisch von Lyseenzymen im Wesentlichen aus wenigstens einem Enzym, ausgewählt aus der Gruppe bestehend aus Lysozym, Lysostaphin und Mutanolysin, und wenigstens einem Enzym, ausgewählt aus der Gruppe bestehend aus Lyticase und Zymolase, besteht.

5. Verfahren zum mikrobiellen Zellaufschluss nach einem der vorhergehenden Ansprüche, wobei die Inkubation zwischen etwa 25°C und etwa 37°C über einen Zeitraum zwischen etwa 10 Minuten und etwa 60 Minuten erfolgt.

6. Verfahren zum mikrobiellen Zellaufschluss nach einem der vorhergehenden Ansprüche, wobei der Chelatbildner und das Gemisch von Lyseenzymen gleichzeitig zu der Probe gegeben werden.

7. Verfahren zum mikrobiellen Zellaufschluss nach einem der vorhergehenden Ansprüche, wobei es sich bei dem Chelatbildner um EDTA handelt.

8. Verfahren zum mikrobiellen Zellaufschluss nach einem der vorhergehenden Ansprüche, bei dem man ferner eine proteasehaltige lysierte mikrobielle Probe durch Zugabe einer Protease zu der Flüssigkeitsprobe oder Suspension herstellt.

9. Verfahren zum mikrobiellen Zellaufschluss nach Anspruch 8, bei dem man ferner
die proteasehaltige lysierte mikrobielle Probe bei einer Temperatur zwischen etwa 50°C und etwa 65°C inkubiert.

10. Verfahren zum mikrobiellen Zellaufschluss nach Anspruch 8 oder Anspruch 9, wobei die Protease zu der lysierten mikrobiellen Zellprobe gegeben wird.

11. Verfahren zum mikrobiellen Zellaufschluss nach Anspruch 8, 9 oder 10, wobei die Protease zur gleichen Zeit wie das Gemisch von Lyseenzymen zu der Flüssigkeitsprobe oder Suspension gegeben wird.

12. Verfahren zum mikrobiellen Zellaufschluss nach Anspruch 8, 9, 10 oder 11, wobei es sich bei der Protease um Proteinase K handelt.

13. Verfahren zum mikrobiellen Zellaufschluss nach einem der Ansprüche 1-12, bei dem man in einem weiteren Schritt die freigesetzten mikrobiellen Nukleinsäuren isoliert.

14. Verfahren nach Anspruch 13, wobei der Isolierungsschritt die Zugabe eines Chaotrops, eines Detergens und eines Nukleinsäure bindenden Trägers zu der lysierten mikrobiellen Zellprobe unter Bedingungen, die die Bindung der freigesetzten Nukleinsäure an den Nukleinsäure bindenden Träger gestatten, umfasst.

15. Verfahren nach einem der Ansprüche 1-14, wobei die Probe ausgewählt ist aus der Gruppe bestehend aus einer Blutprobe, einer konzentrierten Probe roter Blutzellen, einer Thrombozytenprobe, einer Plasmaprobe, einer Serumprobe, einer Urinprobe, einer Speichelprobe, einer Rückenmarksflüssigkeitsprobe, einer interstitiellen Flüssigkeitsprobe, einer Gewebebiopsieprobe, einer Lavagenprobe, einer Sputumprobe sowie einer vaginalen, dentalen, rektalen, Uterus- oder Inertoberfläche-Abstrichprobe.

## Revendications

1. Procédé de rupture de cellules microbiennes pour permettre la libération d'un acide nucléique à partir de cellules microbiennes présentes dans un échantillon, comprenant :
la mise à disposition d'un échantillon contenant, ou que l'on suspecte de contenir, des cellules microbiennes, l'échantillon étant un échantillon liquide ou une suspension,
la production d'une composition pour lyse de l'échantillon, (1) par addition d'un agent chélatant à l'échantillon jusqu'à une concentration finale comprise entre environ 0,3 M et 0,5 M, (2) par addition à l'échantillon d'un mélange d'enzymes de lyse,
et l'incubation de l'échantillon pendant un laps de temps suffisant et à une température permettant de produire un échantillon de cellules microbiennes lysé, de façon à permettre de ce fait la libération de l'acide nucléique microbien.

2. Procédé de rupture de cellules microbiennes selon la revendication 1, dans lequel ledit agent chélatant est choisi dans le groupe consistant en l'EDTA et l'EGTA et leurs sels.

3. Procédé de rupture de cellules microbiennes selon l'une quelconque des revendications précédentes, dans lequel ledit mélange d'enzymes de lyse comprend au moins une enzyme choisie dans le groupe consistant en le lysozyme, la lysostaphine et la mutanolysine, et au moins une enzyme choisie dans le groupe consistant en la lyticase et la zymolase.

4. Procédé de rupture de cellules microbiennes selon l'une quelconque des revendications précédentes, dans lequel ledit mélange d'enzymes de lyse consiste essentiellement en au moins une enzyme choisie dans le groupe consistant en le lysozyme, la lysostaphine et la mutanolysine, et au moins une enzyme choisie dans le groupe consistant en la lyticase et la zymolase.

5. Procédé de rupture de cellules microbiennes selon l'une quelconque des revendications précédentes, dans lequel ladite incubation est effectuée à une température comprise entre environ 25°C et environ 37°C pendant d'environ 10 minutes à environ 60 minutes.

6. Procédé de rupture de cellules microbiennes selon l'une quelconque des revendications précédentes, dans lequel ledit agent chélatant et ledit mélange d'enzymes de lyse sont ajoutés à l'échantillon en même temps.

7. Procédé de rupture de cellules microbiennes selon l'une quelconque des revendications précédentes, dans lequel ledit agent chélatant est l'EDTA.

8. Procédé de rupture de cellules microbiennes selon l'une quelconque des revendications précédentes, qui comprend en outre la réalisation d'un échantillon microbien lysé contenant une protéase, par addition d'une protéase audit échantillon liquide ou à ladite suspension.

9. Procédé de rupture de cellules microbiennes selon la revendication 8, qui comprend en outre l'incubation dudit échantillon microbien lysé, contenant une protéase, à une température comprise entre environ 50°C et environ 65°C.

10. Procédé de rupture de cellules microbiennes de la revendication 8 ou 9, dans lequel ladite protéase est ajoutée audit échantillon de cellules microbiennes lysé.

11. Procédé de rupture de cellules microbiennes de la revendication 8, 9 ou 10, dans lequel la protéase est ajoutée audit échantillon liquide ou à ladite suspension en même temps que ledit mélange d'enzymes de lyse.

12. Procédé de rupture de cellules microbiennes de la revendication 8, 9, 10 ou 11, dans lequel ladite protéase est la protéinase K.

13. Procédé de rupture de cellules microbiennes de l'une quelconque des revendications 1 à 12, comprenant l'étape supplémentaire consistant à isoler lesdits acides nucléiques microbiens libérés.

14. Procédé de la revendication 13, dans lequel ladite étape d'isolement comprend l'addition d'un agent chaotropique, d'un détergent et d'un support pour liaison d'acides nucléiques audit échantillon de cellules microbiennes lysé, dans des conditions qui permettent la liaison desdits acides nucléiques libérés audit support pour liaison d'acides nucléiques.

15. Procédé de l'une quelconque des revendications 1 à 14, dans lequel ledit échantillon est choisi dans le groupe consistant en un échantillon sanguin, un échantillon de globules rouges concentrés, un échantillon de plaquettes, un échantillon de plasma, un échantillon de sérum, un échantillon d'urine, un échantillon de salive, un échantillon de liquide rachidien, un échantillon de fluide interstitiel, un échantillon de biopsie tissulaire, un échantillon de lavage, un échantillon de crachat, et un échantillon de tampon vaginal, dentaire, rectal, utérin ou de surface inerte.
